# EUROPEAN PATENT APPLICATION

(11) **EP 4 782 533 A1**
(43) Date of publication of application: **29.07.2026**
(21) Application number: 24867475.6
(22) Date of filing: 19.09.2024
(51) Int. Cl.: C12N 5/00, C08L 89/00, C08L 5/00

(54) **HIGH-SURFACE-AREA AND VIRTUAL-SPHERE MICROCARRIER FOR CELL CULTURE AND FORMATION METHOD THEREFOR**

(30) Priority: 21.09.2023 CN 202311223092
(71) Applicant: Tantti Laboratory Inc., Taoyuan City 33846 (TW)
(72) Inventor: CHEN, Hui, Taoyuan City 33846 (TW); SHEU, Min-shyan, Taoyuan City 33846 (TW); CHEN, Hong-Sheng, Taoyuan City 33846 (TW); SU, Ting-wei, Taoyuan City 33846 (TW); CHIU, Chun-yi, Taoyuan City 33846 (TW); HUNG, Chen-yu, Taoyuan City 33846 (TW); WANG, Shih-yu, Taoyuan City 33846 (TW); CHOU, Bruce C.S., Taoyuan City 33846 (TW)
(74) Representative: Arnold & Siedsma
(86) International application number: PCT/CN2024/119559
(87) International publication number: WO 2025/061069

(57) **Abstract**

Provided is a microcarrier for cell culture prepared from biocompatible macromolecules, wherein a plurality of pit structures are formed on the outer surface of the microcarrier, thereby providing a surface with an increased surface area as an attachment surface for cells; and the roughness of the outer surface of the microcarrier is increased, thereby promoting stable attachment of cells. Also provided is a method for manufacturing the microcarrier for cell culture. The method involves manufacturing a hydrogel particle containing biocompatible macromolecules and having a smooth outer surface, and forming a plurality of pit structures on the outer surface of the hydrogel particle by means of a concaving process.

## Description

### Technical Field

The present invention relates to a cell microcarrier manufactured from a biocompatible macromolecule, the outer surface of which is formed with a plurality of pit structures, which facilitates cell attachment and promotes cell proliferation. The present invention also relates to a method for manufacturing the microcarrier for cell culture.

### Background Art

Traditionally, ex vivo cell culture is performed by attaching cells to tissue culture plastic vessels or extracellular matrix attachment proteins, followed by administration of appropriate liquid media to promote growth and proliferation. However, this two-dimensional plane culture is far from the actual physiological environment in vivo, and cannot simulate the interaction of cells with extracellular matrix and cells with each other in vivo, nor is it conducive to the reproduction of complex cellular behaviors such as cell migration, apoptosis, transcriptional regulation and receptor expression. The two-dimensional culture method further limits the growth space of cells, which is not conducive to the mass production of cells. Three-dimensional (3D) cell culture technology is obviously a better solution to address the aforementioned industrial challenges. Microcarriers or scaffolds are materials often used in three-dimensional cell culture technology, and they usually have non-toxic, non-rigid and uniform-density properties. It enables adherent cells to attach and grow on the surface of microcarrier particles during dynamic culture. Microcarriers typically have a large surface area-to-volume ratio, resulting in higher cell yield per unit volume of culture medium compared with static, planar culture techniques.

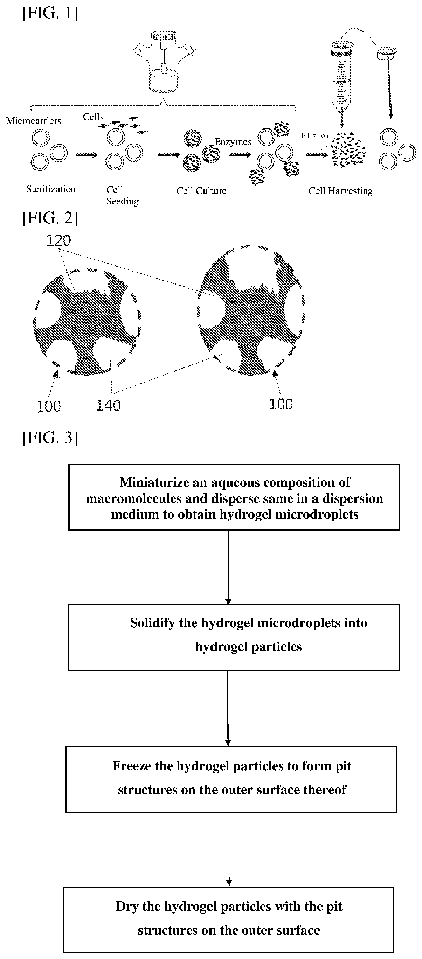

The microcarriers commonly used in laboratories and industry are the Cytodex^{®} series produced by Cytiva. Their main material uses cross-linked dextran as the core, and the surface is modified or coated with materials such as DEAE to make them suitable for cell attachment and growth. FIG. 1 shows the process of using this type of microcarrier to produce cells, in which microcarriers are placed in a bioreactor and sterilized, then inoculated with the required cells, and the culture medium is injected and periodically replaced for cell culture. To terminate cell culture, reagents are used to separate cells from microcarriers, followed by complete separation via filtration, and the harvested cells can be used for subsequent applications such as cell transplantation. However, this conventional microcarrier has significant drawbacks in application. Due to the cross-linked form of dextran microcarriers, they are difficult to dissolve in the tissues where the cultured cells are transplanted later, so it is necessary to detach the cultured cells from the microcarriers in the later stage of the culture process before use. Furthermore, when harvesting cells, enzymes such as trypsin are often used to break the bonds between cells and microcarrier materials. **In** addition to increasing raw material costs during production, such a culture process also consumes valuable time. The use of trypsin cannot achieve 100% separation of cells from microcarriers, and the separated cells cannot survive with 100% viability, so the overall cell recovery rate can only reach about 70%. Finally, when separating microcarriers from cells by methods such as filtration, filter blockages often occur. To completely wash off the cells on the microcarriers, filters must be frequently replaced and large amounts of buffer solution used for cleaning, which is cumbersome and requires a large volume of solvents. Due to the high cost and procedural complexity, the aforementioned conventional three-dimensional (3D) cell culture technology does not meet the requirements of large-scale cell culture.

In particular, in recent years, the development of the artificial meat industry through cell culture has been quite rapid. It not only helps solve the food problem, but also serves as an important technology for reducing environmental pollution and greenhouse gas emissions, while also accommodating the non-killing requirements of specific religions. For people who adopt a vegetarian lifestyle out of love for animals, artificial meat provides them with an opportunity and choice to start eating meat again. However, common but inedible polystyrene cannot be used in the manufacture of cultured meat, nor can microcarriers or scaffolds made from materials of animal origin such as gelatin be used. Therefore, there is a particular need to develop microcarriers or scaffolds made from non-animal-sourced materials.

### Summary of the Invention

To address the aforementioned problems and drawbacks, the present invention provides a microcarrier for cell culture, which is made of biocompatible macromolecules-for example, edible macromolecules or materials that can be dissolved under gentle conditions without harming cells during subsequent cell culture or transplantation. It is thus suitable for various industrial applications requiring large-scale cell production, including artificial meat production. Moreover, in the later stage of cell culture, the microcarrier of the present invention does not require separation of the cultured cells from the microcarrier, thus achieving an almost 100% recovery rate. More importantly, the microcarrier of the present invention has a granular configuration, with an irregular but smooth outer surface without sharp edges, which is referred to herein as a virtual-sphere microcarrier. This appearance configuration is formed by manufacturing hydrogel particles containing the aforementioned microcarrier material and having a smooth outer surface, and forming a plurality of pit structures on the outer surface of the hydrogel particles via a concaving process. The pit structures on the outer surface of the microcarrier not only provide an increased surface area as an attachment surface, making the cells accessible and attachable, but they also increase the roughness of the microcarrier's outer surface, promoting stable cell attachment and thus providing a culture environment conducive to cell growth and proliferation.

Accordingly, a first aspect of the present invention provides a virtual-sphere microcarrier for cell culture, comprising:
a continuous medium composed of one or more biocompatible macromolecules, which has an irregular particle morphology and a particle size in the range of 200 µm to 1000 µm; wherein the continuous medium has an outer surface, and a plurality of pit structures are formed on the outer surface; and the pit structures are prepared by subjecting a solid sphere composed of the one or more biocompatible macromolecules to a freezing process.

A second aspect of the present invention provides a method for manufacturing the aforementioned microcarriers, comprising the steps of:
A) Miniaturizing an aqueous composition comprising one or more biocompatible macromolecules and dispersing it in a dispersion medium to obtain a plurality of hydrogel microdroplets dispersed in the dispersion medium and having smooth outer surfaces;
B) Solidifying the hydrogel microdroplets into hydrogel particles and separating the hydrogel particles from the dispersion medium.
C) Freezing the hydrogel particles at a freezing temperature between -10°C and -60°C and for a freezing time between 8 hours and 24 hours, so that a plurality of pit structures are formed on the outer surface of the hydrogel particles; and
D) Drying the hydrogel particles formed with pit structures to obtain microcarriers in the form of continuous media exhibiting irregular particle morphologies and having a particle size of between 200 microns and 1000 microns.

A third aspect of the present invention provides another method for manufacturing the aforementioned microcarriers, comprising the steps of:
A) Miniaturizing an aqueous composition comprising one or more biocompatible macromolecules and dispersing it in a dispersion medium in the presence of a pore former to obtain a plurality of hydrogel microdroplets dispersed in the dispersion medium and having smooth outer surfaces.
B) Solidifying the hydrogel microdroplets into hydrogel particles and removing the pore former, so that a plurality of pit structures are formed on the outer surface of the hydrogel particles, and separating the hydrogel particles from the dispersion medium; and
C) Drying the hydrogel particles formed with pit structures to obtain microcarriers in the form of continuous media exhibiting irregular particle morphologies and having a particle size between 200 microns and 1000 microns.

### Description of Drawings

FIG. 1 shows the process of using conventional microcarriers to produce cells;
FIG. 2 is a schematic diagram of a microcarrier according to one embodiment of the invention;
FIG. 3 is a flow chart of the first method of the invention for manufacturing microcarriers;
FIG. 4 is an optical electron microscopy image according to one embodiment of the present invention, wherein FIG. 4a shows hydrogel microdroplets containing biocompatible macromolecules dispersed in the dispersion medium and exhibiting a spherical shape; FIG. 4b shows hydrogel particles with pit structures formed thereon; FIG. 4c shows the microcarriers obtained after drying, where the dashed lines represent the original spherical configuration before drying, and the double arrows indicate the geometric dimensions measured under an electron microscope;
FIG. 5 is a flow chart of the second method of the invention for manufacturing microcarriers; and
FIG. 6 shows the cell growth curves obtained by culturing cells with the microcarriers of the present invention, ground irregular microcarriers and Unitantrix^{®} microcarriers.

List of Reference Numerals:
Microcarrier 100
Continuous Medium 120
Pit Structure 140.

### SPECIFIC EMBODIMENTS

FIG. 2 is a schematic diagram of two examples of the present application, showing that the microcarrier 100 mainly comprises a continuous medium 120. As used in this specification, "continuous medium" refers to a single-piece medium consisting of one or more biocompatible macromolecules, suitable for cell attachment, growth, proliferation and migration. The term "biocompatibility" as used herein refers to a material property that does not cause adverse effects on biological systems such as cells, tissues and organs. Biocompatible macromolecules suitable for making microcarriers of the present application include, but are not limited to: Proteins such as gelatin, collagen, fibrins and the like; polysaccharides such as konjac glucomannan (KG), agarose, hyaluronic acid, chitin, alginate, cellulose, gellan gum, carrageenan, dextran, guar gum, xanthan gum and the like; synthetic macromolecules including biodegradable macromolecules such as polyesteramides, polycaprolactone polyols (PCL), polylactic acid (PLA), polyglycolic acid (PGA), polylactic-co-glycolic acid copolymers (PLGA), as well as non-biodegradable macromolecules such as polydimethylsiloxane (PDMS), thermoplastic polyurethane, polyethylene terephthalate (PET), polytetrafluoroethylene (PTFE), polyvinylidene fluoride (PVDF), polystyrene and the like; and combinations thereof. In a preferred embodiment, the biocompatible macromolecule is selected from edible macromolecules. The term "edible" as used herein refers to a material that can be ingested orally by animals, preferably mammals, and more preferably humans, without causing harm or adverse side effects to biological systems such as cells, tissues and organs. Edible macromolecules suitable for use in the present invention include, but are not limited to: Plant-based polysaccharides such as konjac glucomannan, agarose, alginates, cellulose, gellan gum, carrageenan, dextran, guar gum, xanthan gum, starch, and the like; animal-based polysaccharides such as hyaluronic acid, chitin, and the like; plant-based proteins such as pea protein (PP), soy protein, lentil protein, chickpea protein and other legume proteins; peanut protein, almond protein, hemp seed protein, chia seed protein and other nut proteins; quinoa protein, wheat protein, rice protein, corn protein and other cereal proteins; potato protein, vegetable crop protein, fungal protein, algal protein, and the like; animal-based proteins such as gelatin, collagen, fibrin and the like; and combinations thereof. In a preferred embodiment, the edible macromolecules is selected from macromolecules that can be dissolved during subsequent cell culture or transplantation, so as to disintegrate the microcarriers by dissolving the macromolecule in the subsequent process, thereby releasing the cells attached to the microcarriers and harvesting them. These dissolvable macromolecules include, but are not limited to, alginates, cellulose, gellan gum, dextran, guar gum, xanthan gum, and starch.

In a preferred embodiment, the microcarriers of the present invention are basically made of plant-based polysaccharides, such as konjac glucomannan (KG). KG is a polysaccharide macromolecule composed of glucose and mannose linked by β-1,4-glycosidic bonds, which can be extracted from Amorphophallus konjac. KG has good intermolecular forces and can easily form stable, robust solids. KG also has characteristics such as high hydrophilicity, a large number of active hydroxyl groups, carboxyl functional groups, and resistance to non-specific adsorption with biomacromolecules, which makes cells easily accessible and adherent, and thus well suited as a microcarrier material for cell culture. Microcarriers made with KG as the primary material have good processability for controlling the structure of the final product, and their structural integrity can be maintained during one month of cell culture in cell culture medium, demonstrating their ability to withstand structural damage caused by mechanical forces or fluid shear in the culture system. KG is one of the sources of dietary fiber in food, with favorable food safety profiles, low toxicity and minimal health risks. KG also possesses biodegradable properties, as natural degrading enzymes exist in the environment to break it down, preventing long-term environmental persistence.

In a more preferred embodiment, the raw materials for the microcarriers of the present invention, in addition to plant polysaccharides such as KG, also include plant-based proteins (e.g., pea protein) to provide suitable surface chemistry and enhance the adhesion of cells to the microcarrier surface. For example, pea protein features well-established purification and mass production technologies. It can be used as a primary protein source in formulations such as those containing whey or for high-protein fitness, and has a reliable track record in terms of food safety.

In another more preferred embodiment, the microcarriers of the present invention are made from raw materials including KG and a small amount of alginate, so as to adjust the solution-colloid transition rate of KG by controlling the crosslinking rate of alginate, thereby preventing premature gelation of KG during solution preparation.

As shown in FIG. 2, the microcarrier 100 of the present invention exhibits a particulate morphology and has an irregular yet smooth outer surface without sharp edges. As described hereinafter, the microcarriers of the present invention originally had a spherical configuration as indicated by the dashed lines; this configuration is not limited to perfect spheres (as shown on the left of FIG. 2), but also includes near-spherical shapes, ellipsoids (as shown on the right of FIG. 2) and other smooth-curved forms. By means of a concaving process, a plurality of pit structures 140 are formed on the smooth outer surface of the original sphere (as indicated by the dotted line), resulting in irregularly shaped particles. The microcarriers of the present invention are therefore referred to as virtual-sphere microcarriers. As used herein, the term "irregular" means that the microcarrier deviates from its original spherical configuration to a certain degree. According to the present invention, the particle size of the microcarrier 100 is in the range of 200 µm to 1000 µm, preferably in the range of 250 µm to 800 µm, for example in the range of 300 µm to 700 µm, depending on the type of cells to be cultured and the culture conditions. The particle size of the microcarrier 100 can be determined by optical or electron microscopy or other measurement techniques, which will not be elaborated herein. The distribution of the pit structures 140 on the outer surface of the microcarrier may be ordered, disordered, or partially ordered, while the pit structures 140 may be interconnected, overlapping, or discrete. The appearance of the individual pit structures 140 may be smooth curved surfaces, irregular rough surfaces, or a combination of the two. The smallest geometric dimension of the individual pit structures 140 is in the range of 50 µm to 300 µm, more preferably in the range of 100 µm to 250 µm, for example in the range of 100 µm to 200 µm. As used herein, the term "minimum geometric scale" refers to the smallest dimension used to describe the appearance of an individual pit structure, such as the smallest among the side length, depth, diameter and other such dimensions of pit structure 140 measured using electron microscopy. As described hereinafter, the size and number of pit structures can be adjusted by controlling the parameters and conditions of the concaving process.

By forming pit structures, the microcarriers of the present invention can effectively increase their surface area-to-volume ratio, thereby increasing the cell attachment area and cell growth efficiency. These pit structures also increase the surface roughness of the microcarriers, thereby enhancing cellular adhesion during attachment and growth, which makes the cells less likely to detach due to rapid flow or agitation of the culture medium during dynamic culture. These characteristics render the microcarriers of the present invention suitable for industrial applications including artificial meat, in vitro organoid culture, artificial skin, artificial leather, cell therapy, gene therapy and the like, without being limited thereto.

FIG. 3 is a flow chart of the first method for manufacturing microcarriers of the present invention, which involves using freezing as a means of forming pit structures on the outer surfaces thereof after curing hydrogel microdroplets containing biocompatible macromolecules. The method comprises Step A: Miniaturizing and dispersing an aqueous composition comprising biocompatible macromolecules in a dispersion medium to yield a plurality of hydrogel microdroplets; Step B: Solidify the hydrogel microdroplets into hydrogel particles; Step C: Freeze the hydrogel particles to form pit structures on the outer surface thereof; and Step D: Dry the hydrogel particles with the pit structures on the outer surface to obtain the virtual-sphere microcarriers of the present invention.

**In** Step A, components including biocompatible macromolecules and other ingredients may first be dissolved in an aqueous solvent to form an aqueous composition, and a dispersion medium may be prepared. The aqueous composition is then miniaturized and dispersed in the dispersion medium. **In** preparing the aqueous composition, dissolution may be facilitated by such means as heating, cooling, agitation, homogenization and ultrasonication. **In** embodiments where the biocompatible macromolecules have high viscosity, a high-speed homogenizer may be employed to impart high shear forces to the aqueous composition to promote uniform mixing of the components.

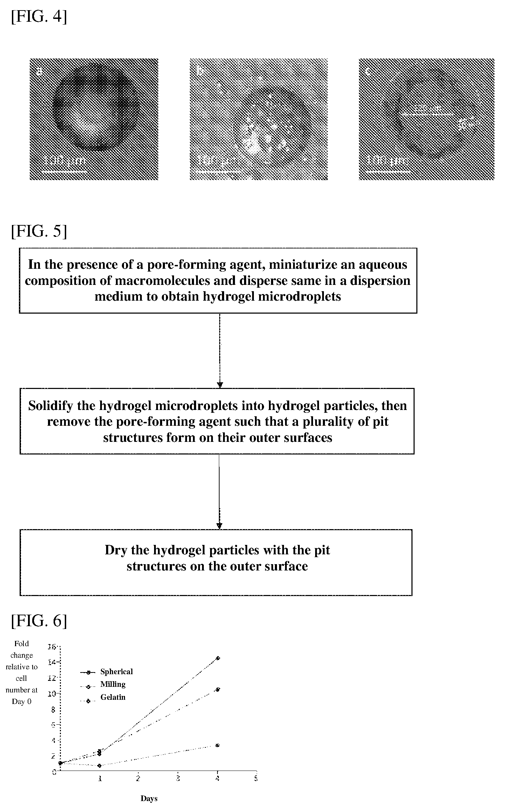

**In** a preferred embodiment, the dispersion medium comprises an oily liquid that is immiscible with the aqueous composition, and the miniaturization and dispersion process comprises emulsifying the aqueous composition with the dispersion medium to produce a water-in-oil emulsion. **In** the emulsification process, the aqueous composition is miniaturized and dispersed in the dispersion medium serving as the continuous phase, wherein the dispersed phase comprises a plurality of hydrogel microdroplets. **In** another preferred embodiment, the dispersion comprises an aqueous solvent, and the miniaturization and dispersion process comprises miniaturizing the aqueous composition into a plurality of hydrogel microdroplets and dispersing them in the aqueous solvent. The means for miniaturization and dispersion include, but are not limited to, dropwise addition, spraying, membrane emulsification, mechanical agitation, high-speed shearing, and combinations thereof. The size of the hydrogel microdroplets can be adjusted by controlling the parameters and conditions of each dispersion method. For example, in embodiments where mechanical agitation or high-speed shearing is used as the means of miniaturization and dispersion, the size of the hydrogel microdroplets may be adjusted by controlling conditions such as the agitation speed and temperature of the stirrer or homogenizer and/or the viscosity of the dispersion medium. In embodiments where spraying or membrane emulsification is used as the means of miniaturization and dispersion, the size of the hydrogel microdroplets may be adjusted by selecting the nozzle size or the pore size of the emulsification membrane, respectively. In a more preferred embodiment, the present invention uses high-speed shearing as the means of miniaturization and dispersion. The inventors of the present invention have found that high-speed shearing can overcome the problem in which high-viscosity aqueous compositions are difficult to pass through narrow pores when dropwise addition, spraying or membrane emulsification is used as the means of miniaturization and dispersion. Thus, the content of biocompatible macromolecules in the aqueous composition can be effectively increased, thereby enhancing the mechanical strength of the final microcarrier products and endowing them with higher structural stability during long-term immersion and agitation in the culture medium. The hydrogel microdroplets dispersed in the dispersion medium will spontaneously form substantially perfect spheres, near-spherical shapes or ellipsoids due to their intrinsic cohesion, and thus have smooth outer surfaces, as shown in FIG. 4a.

As used herein, the term "aqueous solvent" refers to a primarily water-based solvent system. Suitable aqueous solvents refer to any known aqueous solvents capable of solubilizing biocompatible macromolecules without undergoing substantial chemical reactions with the biocompatible macromolecules or other components in the manufacturing process; preferably, the aqueous solvent itself is biocompatible, and more preferably, the aqueous solvent contains no components of animal origin. These solvents include but are not limited to water such as deionized water and pure water, aqueous solutions of C₁₋₄ alcohols such as 50-95% by volume aqueous ethanol solution, aqueous solutions of salts, acids and bases such as aqueous solutions of sodium chloride, calcium chloride, acetic acid, sodium acetate, sodium bicarbonate, sodium carbonate, sodium phosphate, disodium hydrogen phosphate, sodium dihydrogen phosphate, sodium trimetaphosphate and combinations thereof, aqueous solutions of sugars including linear or cyclic monosaccharides, disaccharides and polysaccharides such as aqueous solutions of glyceraldehyde, erythrose, arabinose, xylose, fructose, glucose, galactose, mannose, sedoheptulose, lactose, sucrose, maltose, trehalose, starch, glycogen, cellulose, chitin and combinations thereof, aqueous solutions of sugar alcohols such as aqueous solutions of glucitol, xylitol, sorbitol, maltitol, erythritol, lactitol, mannitol and combinations thereof, aqueous solutions of polyols and syrups such as aqueous solutions of glycerol, maple syrup, corn syrup, pomegranate syrup, as well as aqueous solutions of polyethers such as aqueous polyethylene glycol solutions. Preferably, the aqueous solvent is selected from the group consisting of water and salt solutions thereof.

The aforementioned "oily liquids" are preferably biocompatible, and more preferably oils of non-animal origin, including but not limited to silicone oils, sunflower oil, canola oil, salad oils, olive oil, and combinations thereof.

A surfactant may be optionally included in the aqueous composition and/or dispersion medium for facilitating the dispersion of materials and/or stabilizing the water-in-oil emulsion, so that the hydrogel microdroplets dispersed in the continuous phase do not merge with each other. Surfactants suitable for use in the present invention include, but are not limited to, cationic surfactants, e.g., fatty ammonium salts, ethanolammonium salts, polyethylene polyammonium salts; anionic surfactants, e.g., alkylbenzene sulfonates, alkyl sulfonate esters, alkyl sulfonates, alkyl sulfates, alkenyl sulfonates, naphthenates, stearates; zwitterionic surfactants, e.g., alkyl amino acids, carboxybetaines, sulfobetaines, phosphate betaines, alkyltetrahydroimidazoles, polyacrylamides; and nonionic surfactants, e.g., fatty alcohol ethoxylates, alkylphenol ethoxylates, fatty acid polyoxyethylene esters, alkyl ethoxyamines, alkyl polyoxyethylene amides, polyethers. Preferably, the surfactant is present in the emulsion at a concentration of about 0 to 30 wt%.

In the embodiment where the dispersion medium is an oily liquid, the aqueous composition and/or the dispersion medium may optionally contain solid particles that can facilitate emulsion stabilization, so that the water-in-oil emulsion produced in Step A becomes a Pickering emulsion, thereby preventing the coalescence of the hydrogel microdroplets dispersed in the continuous phase. Solid particles suitable for use in the present invention include, but are not limited to, silica and polystyrene.

Step B involves curing the hydrogel microdroplets dispersed in the dispersion medium to form hydrogel particles, so that their structures have sufficient mechanical strength to withstand the subsequent concaving process without disintegration. As used herein, the term "curing" refers to the process of applying physical or chemical crosslinking methods to hydrogel microdroplets in a fluid state, thereby converting them into hydrogel particles with a stable structure. The reaction conditions for curing vary depending on the type of biocompatible macromolecule used, but are well known in the art. For example, in the embodiment where konjac glucomannan (KG) is used as the biocompatible macromolecule, the temperature can be increased to gel and set the hydrogel microdroplets. In embodiments where collagen or gelatin is used as the biocompatible macromolecule, the hydrogel microdroplets can be dehydrated at low temperatures to achieve gelation and setting. In embodiments where alginate is used as the biocompatible macromolecule, salts capable of dissociating divalent metal ions (e.g., calcium ions or magnesium ions) can be added to induce crosslinking reactions between the alginate molecules within the hydrogel microdroplets, thereby effecting curing and setting.

Step B further separates the solidified hydrogel particles from the dispersion medium. Suitable separation processes include all conventional solid-liquid separation processes. In a preferred specific example, the hydrogel particles may be separated by filtration methods, such as filtration using a sieve, membrane, vacuum suction, wet filtration, or sieve shaker. The separated hydrogel particles may be washed several times with water (e.g., deionized water, pure water) and/or alcohol-based solvents (e.g., ethanol, isopropanol) to remove residual dispersion medium.

Step C involves a concaving process in which the separated hydrogel particles are frozen. The inventors of the present invention found that freezing temperature is a key condition for determining the size of the pit structures, and that freezing time affects the integrity of the pit structures. If the freezing temperature or freezing time is insufficient, not only will the pit structures be incomplete and their average size be out of control, but also the wall thickness of the pit structures will be uneven, which may constitute structural weak points of the microcarriers and is unfavorable for withstanding shear forces caused by culture medium flow during long-term immersion under cell culture conditions. Therefore, the freezing temperature is selected to be in the range of -10 °C to -60 °C and the freezing time in the range of 8 - 24 hours, preferably with the product of the absolute value of freezing temperature and freezing time ≥ 250 °C • h, e.g., ≥ 300 °C • h. This ensures that the smallest geometric dimension of each individual pit structure is in the range of 50 µm to 300 µm, more preferably 100 µm to 250 µm, e.g., 100 µm to 200 µm. In one embodiment where KG is used as the biocompatible macromolecule, pit structures with diameters in the range of 150 - 200 µm can be formed on the outer surface of the hydrogel particles using a freezing temperature of about -20 °C and a freezing time of about 16 hours. In another embodiment where KG is used as the biocompatible macromolecule, pit structures with diameters in the range of 30 - 80 µm can be formed on the outer surface of the hydrogel particles using a freezing temperature of about -50 °C and a freezing time of about 10 hours. An example of the hydrogel particles obtained after performing Step C is shown in FIG. 4b.

As used herein, the term "freezing" may also encompass lyophilization (freeze-drying), which includes a freezing step to freeze the water within the hydrogel particles into ice crystals, and a drying step to sublimate the ice crystals into water vapor under reduced pressure, which is then removed by evacuation.

In Step D, the hydrogel particles with pit structures formed thereon are dried to remove moisture. In addition to facilitating storage, the dried particles are less prone to developing contaminants and can be easily subjected to processes required for aliquoting, such as weighing and volume measurement. Suitable drying methods are any known drying methods capable of achieving the aforementioned moisture removal objective, including but not limited to hot air drying and freeze-drying. The dried hydrogel particles may undergo slight volume shrinkage, causing their outer surfaces to deform slightly and present as a continuous matrix with irregular particle morphology, as shown in FIG. 4c, wherein the white dashed line indicates the spherical shape prior to the formation of pit structures. Compared with hot air drying, microcarriers dehydrated via freeze-drying exhibit minimal volume shrinkage, which represents a distinct advantage.

Microcarriers prepared via the aforementioned methods have particle sizes in the range of 200 µm to 1000 µm. The dried microcarriers can be rehydrated via pre-immersion in culture medium prior to use in cell culture and be ready for cell inoculation.

FIG. 5 is a flow chart of a second method for manufacturing microcarriers of the present invention, comprising Step A:In the presence of a pore-forming agent, an aqueous composition comprising biocompatible macromolecules is miniaturized and dispersed in a dispersion medium to obtain a plurality of hydrogel microdroplets; Step B:Solidify the hydrogel microdroplets into hydrogel particles to form pit structures on their outer surfaces; and Step C:Dry the hydrogel particles with the pit structures on the outer surface to obtain the virtual-sphere microcarriers of the present invention.

The definitions of chemical materials and processing methods set forth in the aforementioned second method are the same as those defined for the first method above. The main difference between the second method and the first method of the present invention lies in the timing and means of forming pit structures; that is, the second method forms pit structures by adding a pore-forming agent before the hydrogel microdroplets are solidified, whereas the first method achieves this by freezing the hydrogel microdroplets after solidifying thereof. According to the present invention, the pore-forming agent may be added to the aqueous composition or the dispersion medium in Step A. Preferably, the pore-forming agent is incorporated into the resultant aqueous composition via means such as aeration, stirring, homogenization, shaking and membrane emulsification, subsequent to the dissolution of the biocompatible macromolecule in an aqueous solvent to form the aqueous composition. The pore-forming agent is then distributed into the hydrogel microdroplets, which remain in a fluid state, during the miniaturization of the aqueous composition. At this stage, numerous pore-forming agent molecules or gases derived therefrom will be present near the outer surfaces of the hydrogel microdroplets and occupy void spaces, causing the outer surfaces of the hydrogel microdroplets to indent inward. Accordingly, pit structures are formed as the hydrogel microdroplets are solidified into hydrogel particles in Step B. The pore-forming agents include, but are not limited to, gases, foaming agents, oily liquids, and aqueous solvents containing surfactants.

In Step B of the second method of the present invention, the process may further include a step of removing the pore-forming agent subsequent to the curing process. In one example, the pore-forming agent may be removed in conjunction with the drying process via means such as gas purging, air sparging and vacuum evacuation, thereby leaving the pit structures intact. In embodiments where gas-generating pore-forming agents such as ammonium bicarbonate are employed, the pore-forming agent may be induced to generate gas via physical or chemical means such as heating or pH adjustment during or after the curing of the hydrogel microdroplets, so as to form the pit structures. The generated gas escapes spontaneously or is removed via such means as gas purging, air sparging and vacuum evacuation. In the embodiment where a homogeneous pore-forming agent is used, after the formation of pit structures during the curing of the hydrogel microdroplets, the pore-forming agent is removed via such means as gas purging, air sparging and vacuum evacuation, thereby retaining the pit structures. Microcarriers prepared via this method have a particle size in the range of 200 µm to 1000 µm.

The following examples are for illustrative purposes only and do not limit the scope of the present invention.

### Example 1: Preparation of Hydrogel Particles

A high-speed homogenizer was used to apply shear forces for dissolving konjac glucomannan (KG), calcium alginate (ALG) and biocompatible hardness-enhancing additives in deionized water to prepare an aqueous composition. The aqueous composition was then dispersed in a canola oil dispersion medium using a high-speed homogenizer to form a water-in-oil emulsion. During dispersion, the aqueous composition containing konjac glucomannan is transformed into microdroplets by the sheer force exerted by the high-speed homogenizer, wherein the microdroplet size and dispersion uniformity can be controlled by adjusting parameters such as the rotation speed of the homogenizer and the viscosity of the dispersion medium. At this stage, the aqueous composition, which was dispersed into microdroplets, exhibited a smooth, curved surface morphology in the dispersion medium due to surface tension-typically a spherical morphology. After dispersion, the resulting water-in-oil emulsion was heated at 80 °C for 1 hour to solidify the hydrogel microdroplets into particles, so as to enhance their structural integrity. Agitation was maintained at an appropriate speed throughout the heating process to keep the particles in a dispersed state. Subsequently, these particles were separated from the dispersion medium by filtration. Particles that were excessively large or small could be further removed by sieving with sieves of specific mesh sizes, with an overall recovery rate of 30-95%. During the filtration process, water and alcohol-based solvents could be used for washing to remove substances such as canola oil and surfactants, followed by a final rinse with water.

### Example 2: Preparation of Hydrogel Particles

The powders of KG, ALG and pea protein (PP) were mixed with deionized water, and shear forces were applied to facilitate dissolution, so as to prepare an aqueous composition. The aqueous composition was then rapidly added to the canola oil under continuous agitation in a homogenizer assembly to form a water-in-oil emulsion, in which the dispersed phase microdroplets had a particle size of approximately 300 ± 200 µm. The aqueous calcium chloride solution was then added to the water-in-oil emulsion, followed by heating to an elevated temperature and continuous agitation to impart crosslinking strength to the micronized particles. If a positively charged crosslinking agent is required, it may be added to the aqueous calcium chloride solution and then co-added to the water-in-oil emulsion. The crosslinked water-in-oil emulsion was passed through a sieve of the desired size to isolate hydrogel particles of the desired size. During filtration, canola oil could be removed by washing with water and alcohol-based solvents, followed by a final rinse with water.

### Example 3: Preparation of Hydrogel Particles

The powders of KG and ALG were mixed with deionized water, and shear forces were applied at 80 °C to facilitate dissolution, so as to prepare an aqueous composition. Subsequently, the aqueous composition was dispersed via syringe dropwise addition into water containing calcium ions, whereupon its droplets were converted from a liquid state to a colloidal state, thereby forming spheroidal hydrogel particles containing KG. The particles were sieved to isolate hydrogel particles of the desired size.

### Example 4: Preparation of Microcarriers

The KG-containing hydrogel particles prepared in Example 1 were frozen to form pit structures on their outer surfaces. The hydrogel particles thus treated were then dried to obtain the microcarriers.

### Example 5: Cell Growth Performance of Microcarriers

The same amount of cells was inoculated onto three types of microcarriers, namely the microcarriers prepared in Example 4, irregular microcarriers fabricated from the same KG material in bulk form and then pulverized into particles, and the gelatin-based Unitantrix^{®} microcarriers produced by TANTTI LABORATORY INC., and cultured under conditions optimal for cell growth. Cells were then counted on Day 1 and Day 4 after inoculation. FIG. 6 shows the cell growth curves obtained with the three aforementioned microcarriers. The results showed that cells cultured on the virtual-sphere microcarriers prepared in Example 4 increased to 14 times the initial cell number after 4 days of culture. In contrast, cells cultured on the irregular microcarriers of the same material reached a 10-fold increase after 4 days of culture. Cells cultured on the Unitantrix^{®} microcarriers exhibited only an approximately 3-fold increase in cell number after 4 days. The results of this example indicate that the surface structure of the virtual-sphere microcarriers of the present invention provides a larger surface area and optimal surface roughness, which facilitates the stable adhesion of cells, thereby creating a more favorable environment for cell growth and is also suitable for dynamic culture.

## Claims

1. A virtual-sphere microcarrier for cell culture, **characterized in that** it comprises:
a continuous medium composed of one or more biocompatible macromolecules, which has an irregular particle morphology and a particle size in the range of 200 µm to 1000 µm; wherein the continuous medium has an outer surface, and a plurality of pit structures are formed on the outer surface; and the pit structures are prepared by subjecting a solid sphere composed of the one or more biocompatible macromolecules to a freezing process.

2. The microcarrier of claim 1, wherein the continuous medium has a particle size in the range of 250 µm to 800 µm.

3. The microcarrier of claim 2, wherein the continuous medium has a particle size in the range of 300 µm to 700 µm.

4. The microcarrier of claim 1, wherein each of the pit structures has a minimum geometric dimension in the range of 50 µm to 300 µm.

5. The microcarrier of claim 1, wherein each of the pit structures has a minimum geometric dimension in the range of 100 µm to 250 µm.

6. The microcarrier of claim 1, wherein each of the pit structures has a minimum geometric dimension in the range of 100 µm to 200 µm.

7. The microcarrier of claim 1, wherein the biocompatible macromolecules are selected from the group consisting of plant polysaccharides, animal polysaccharides, plant proteins, animal proteins, and combinations thereof.

8. A method for preparing a microcarrier for cell culture, **characterized in that** it comprises the following steps:
A) miniaturizing and dispersing an aqueous composition comprising one or more biocompatible macromolecules in a dispersion medium to form a plurality of hydrogel microdroplets with smooth outer surfaces dispersed therein;
B) curing said hydrogel microdroplets into hydrogel particles and then separating said hydrogel particles from the dispersion medium;
C) freezing said hydrogel particles at a temperature ranging from -10 °C to -60 °C for a duration of 8 to 24 hours, such that a plurality of pit structures form on the outer surfaces of said hydrogel particles; and
D) drying said hydrogel particles with pit structures formed thereon to obtain microcarriers in the form of a continuous medium exhibiting an irregular particle morphology, said microcarriers having a particle size in the range of 200 µm to 1000 µm.

9. A method for preparing a microcarrier for cell culture, **characterized in that** it comprises the following steps:
A) miniaturizing and dispersing an aqueous composition comprising one or more biocompatible macromolecules in a dispersion medium in the presence of a pore-forming agent to obtain a plurality of hydrogel microdroplets with smooth outer surfaces dispersed therein;
B) curing said hydrogel microdroplets into hydrogel particles, then removing the pore-forming agent such that a plurality of pit structures form on the outer surfaces of said hydrogel particles, and separating said hydrogel particles from the dispersion medium; and
C) drying said hydrogel particles with pit structures formed thereon to obtain microcarriers in the form of a continuous medium exhibiting an irregular particle morphology, said microcarriers having a particle size in the range of 200 µm to 1000 µm.

10. The method of claim 8 or 9, wherein each of the pit structures has a minimum geometric dimension in the range of 50 µm to 300 µm.

11. The method of claim 8 or 9, wherein the miniaturization and dispersion in step A are selected from the group consisting of dropwise addition, spraying, membrane emulsification, mechanical agitation, high-speed shearing, and combinations thereof.

12. The method according to claim 8, wherein the freezing step satisfies the following condition: |Freezing temperature| × Freezing duration ≥ 250 °C·h.

13. The method of claim 8, wherein the freezing step satisfies the following condition: |Freezing temperature| × Freezing duration ≥ 300 °C·h.

14. The method of claim 9, wherein the pore-forming agent is selected from the group consisting of gases, foaming agents, oily liquids, and aqueous solvents containing surfactants.

15. The method of claim 8 or 9, wherein the biocompatible macromolecules are selected from the group consisting of plant polysaccharides, animal polysaccharides, plant proteins, animal proteins, and combinations thereof.

16. The method according to claim 8 or 9, wherein the drying method is selected from the group consisting of hot air drying methods and freeze-drying methods.
